# EUROPÄISCHE PATENTANMELDUNG

(11) **EP 0 737 857 A2**
(43) Veröffentlichungstag der Anmeldung: **16.10.1996**
(21) Anmeldenummer: 96105526.6
(22) Anmeldetag: 06.04.1996
(51) Int. Cl.: G01N 21/89, G01N 33/36

(54) **Vorrichtung zur optischen Überwachung eines Fadens auf Unregelmässigkeiten**

(30) Priorität: 13.04.1995 DE 19514006
(71) Anmelder: Akzo Nobel N.V., NL-6824 BM Arnhem (NL)
(72) Erfinder: Louis, Hans Willi, 52525 Heinsberg (DE); Ketzler, Paul, 52525 Heinsberg (DE)
(74) Vertreter: Fett, Günter

(57) **Zusammenfassung**

Vorrichtung zur optischen Überwachung mindestens eines Fadens auf Unregelmäßigkeiten, insbesondere auf Filamentbrüche von Multifilamentgarnen, mit einer Strahlungsquelle, einem Strahlungsempfänger und einem von der Strahlungsquelle ausgesandten und vom Strahlungsempfänger empfangenen quer zum Faden und am Faden in einem definierten Abstand zum Faden vorbeigeführten Strahlenbündel, wobei die Strahlungsquelle und der Strahlungsempfänger neben dem Faden angeordnet sind, und wobei Strahlungsquelle und Strahlungsempfänger auf derselben Seite des Fadens angeordnet sind, und auf der gegenüberliegenden Seite des Fadens Mittel zum versetzten Rückstrahlen des Strahlenbündels derart angeordnet sind, daß das ausgesandte Strahlenbündel auf der einen Seite des Fadens und das versetzt rückgestrahlte Strahlenbündel auf der anderen Seite des Fadens vorbeigeführt wird, wobei das ausgestrahlte Strahlenbündel und das versetzt rückgestrahlte Strahlenbündel möglichst denselben Abstand zum Faden aufweisen und zumindest in etwa parallel zueinander angeordnet sind.

## Beschreibung

Die Erfindung betrifft eine Vorrichtung zur optischen Überwachung mindestens eines Fadens auf Unregelmäßigkeiten, insbesondere auf Filamentbrüche von Multifilamentgarnen, mit einer Strahlungsquelle, einem Strahlungsempfänger und einem von der Strahlungsquelle ausgesandten und vom Strahlungsempfänger empfangenen quer zum Faden und am Faden in einem definierten Abstand zum Faden vorbeigeführten Strahlenbündel, wobei die Strahlungsquelle und der Strahlungsempfänger neben dem Faden angeordnet sind.

Unregelmäßigkeiten wie Dickenschwankungen, gebrochene Filamente oder Flusen sind an Fäden meist unerwünscht. Vorrichtungen zum Bestimmen der Häufigkeit solcher Unregelmäßigkeiten sind vielfach bekannt. Beispielsweise wird in DE-A-29 00 414 eine oben genannte Vorrichtung beschrieben, bei welcher als Strahlungsquelle eine Lampe eingesetzt wird, die einen Lichtstrahl an dem Faden vorbeisendet, der hinter dem Faden von einem Fototransistor empfangen wird. Weist das Garn Filamentbrüche auf und weisen die deshalb vom Faden abstehenden Filamente in Richtung des Lichtstrahls, wird der Lichtstrahl mehr oder weniger stark abgedunkelt, wodurch Abschattungen vom Fototransistor empfangen werden. Alle Abschattungen, die einen bestimmten, voreinstellbaren Schwellenwert übersteigen, können über eine dem Fototransistor angeschlossene Elektronik gezählt werden. Die Anzahl der Abschattungen wird als Maß für die Haarigkeit des Fadens verwendet. Nachteilig bei diesem Verfahren ist, daß immer nur Filamente, die in einer Richtung aus dem Garn hervorstehen, als Abschattung festgestellt werden können, sodaß nur dann eine klare Aussage über die Haarigkeit des gemessenen Fadens gemacht werden kann, wenn die abstehenden Filamente in etwa gleichmäßig um das Garn herum von diesem abstehen. Hierbei kommt es sehr stark darauf an, daß die Anordnung der zu einer solchen Meßstelle führenden Fadenführer derart angeordnet werden, daß die abstehenden Filamente nicht von dem Fadenführer vor der Meßstelle an den Faden angestrichen werden und somit sich erst nach der Meßstelle wieder aufrichten und deshalb nicht vom Lichtstrahl erfaßt werden. Auch sind aufwendige Justagearbeiten an der bekannten Vorrichtung nicht zu vermeiden, die insbesondere deswegen durchgeführt werden müssen, weil Strahlungsquelle und Strahlungsempfänger auf verschiedenen Seiten des Fadens angeordnet sind. Hierbei müssen auch die Leitungen für die Stromversorgung und die Weiterleitung der empfangenen Signale an verschiedene Stellen innerhalb der Vorrichtung geführt werden. Schließlich ist ein weiterer Nachteil darin zu sehen, daß zwar der Schwellenwert für eine Abschattung um so geringer eingestellt werden kann, je näher am Faden Strahlungsquelle und Strahlungsempfänger angeordnet werden, daß aber gleichzeitig die Staub- und Temperaturempfindlichkeit größer werden.

Dennoch wurde bisher lediglich die Empfindlichkeit der dem Strahlungsempfänger nachgeschalteten Elektronik verbessert, wie es sich aus DE-A-29 33 297, DE-A-43 00 581 und EP-A-0 213 587 ergibt, sodaß auch diese bekannten Vorrichtungen die oben beschriebenen Nachteile aufweisen.

Aufgabe der vorliegenden Erfindung ist es, die bekannten Vorrichtungen zur optischen Überwachung von mindestens einem Faden zu verbessern. Insbesondere soll der Schaltungsaufwand mit der erfindungsgemäßen Vorrichtung vereinfacht werden. In einer bevorzugten Ausführungsform der erfindungsgemäßen Vorrichtung sollen die von Staub und/oder erhöhten Temperaturen entstehenden negativen Einflüsse möglichst weitgehend ausgeschaltet werden.

Diese Aufgabe wird bei einer Vorrichtung der vorgenannten Art dadurch gelöst, , daß Strahlungsquelle und Strahlungsempfänger auf derselben Seite des Fadens angeordnet sind, und daß auf der gegenüberliegenden Seite des Fadens Mittel zum versetzten Rückstrahlen des Strahlenbündels derart angeordnet sind, daß das ausgesandte Strahlenbündel auf der einen Seite des Fadens und das versetzt rückgestrahlte Strahlenbündel auf der anderen Seite des Fadens vorbeigeführt wird, wobei das ausgestrahlte Strahlenbündel und das versetzt rückgestrahlte Strahlenbündel möglichst denselben Abstand zum Faden aufweisen und zumindest in etwa parallel zueinander angeordnet sind. Auf diese Weise können alle Anschlußleitungen, die zur Erfassung von Unregelmäßigkeiten am Faden erforderlich sind, an derselben Stelle angeschlossen werden, weshalb sich die erfindungsgemäße Vorrichtung durch einen einfachen Ausbau auszeichnet.

Bei Fadenbruchdetektoren ist es zwar bekannt, Strahlungsquelle und Strahlungsempfänger auf derselben Seite des Fadens anzuordnen (DE-U-82 33 626), und das Strahlenbündel über einen Reflektor zurückzustrahlen, doch ist die Art des dort verwendeten Reflektors für Vorrichtungen zum optischen Erfassen von Fadenungleichmäßigkeiten, insbesondere von Filamentbrüchen von Multifilamentgarnen nicht geeignet. Bei Fadenbrüchen kommt es nämlich lediglich darauf an, den herunterfallenden Faden optisch zu erfassen. Hierzu reicht eine grobe Ausrichtung aus. Durch das von der Strahlungsquelle, dem Reflektor und dem Strahlungsempfänger gebildete Dreieck wäre selbst durch sehr aufwendige Justierarbeit diese Art der Überwachung nicht für das Zählen von abstehenden Filamenten geeignet. Diese Art der Reflektion hätte also in keiner Weise angeregt, die Vorrichtungen zum optischen Überwachen von Fadenungleichmäßigkeiten einzusetzen. Um Strahlungsquelle, Reflektor und Strahlungsempfänger für die Messung von Fadenungleichmäßigkeiten überhaupt geeignet zu machen, müßten alle Elemente möglichst nah an den Faden herangebracht werden, wodurch die Gefahr der Verstaubung der Elemente stark zunimmt, sodaß eine solche Anordnung schon nach kurzer Zeit unbrauchbar ist und gereinigt werden muß. Insbesondere, wenn Fäden in einer unter erhöhter Temperatur stehenden Athmosphäre untersucht werden müssen, würden infolge von Wärmeausdehnungen der Vorrichtung im Dauerbetrieb das ausgesandte und das rückgestrahlte Strahlenbündel ihren Abstand zum Faden verändern, was zu unbrauchbaren Ergebnissen führt. Um so überraschender ist es, daß durch Mittel, die ein Strahlenbündel versetzt, aber parallel zur Richtung des ankommenden Strahlenbündels zurückreflektieren, eine Reflektionsmethode zur Verfügung gestellt werden kann, die für Vorrichtungen zur Erfassung von Fadenungleichmäßigkeiten geeignet ist.

Im einfachsten Fall sind die Mittel zum versetzten Rückstrahlen des Strahlenbündels zwei in einem Winkel zueinander ausgerichtete Reflektoren, wobei die Reflektoren derart zueinander und zum ankommenden Strahlenbündel angeordnet sind, daß das Strahlenbündel wenige Millimeter, in der Regel in einem solchen Abstand, der geringfügig größer ist als die Dicke des zu messenden Garns, versetzt und parallel zum ankommenden Strahlenbündel rückgestrahlt wird. Bei einer derartigen Vorrichtung, können Strahlungsquelle, die Mittel zum versetzten Rückstrahlen und Strahlungsempfänger in einem größeren Abstand vom Faden positioniert werden, sodaß Staub und Temperatur kaum bis zu den den Verlauf des Strahlenbündels bestimmenden Elementen gelangt. Insofern ist durch die erfindungsgemäße Anordnung eine zuverlässige Erfassung der Menge der Ungleichmäßigkeiten von Multifilamentgarnen auch im Dauerbetrieb möglich.

Die erfindungsgemäße Vorrichtung zeichnet sich insbesondere dadurch aus, daß das Mittel zum versetzten Rückstrahlen des Strahlenbündels ein Umlenk-Prisma ist. Durch den Einsatz von unterschiedlichen Umlenkprismen und durch Nachjustieren von Strahlungsquelle und Strahlungsempfänger läßt sich die erfindungsgemäße Vorrichtung praktisch auf jede Garndicke einstellen.

Für die erfindungsgemäße Vorrichtung eignet sich als Strahlenbündel ein Laserlichtbündel, bevorzugt ein gebündelter Lichtstrahl. Hiebei hat sich besonders ein Lichtstrahl mit einer Wellenlänge bewährt, die im nahen infraroten Bereich liegt. Bekanntlich hat der nahe infrarote Bereich eine Wellenlänge von 780 nm bis 2500 nm.
Derartige Strahlenbündel lassen sich durch an sich bekannte Mittel gut parallelisieren, sodaß sie auch bei Überbrückung größerer Strecken gute Ergebnisse liefern. Zur Erfassung von auch nur geringfügig abstehender Filamente hat es sich besonders bewährt, wenn in Fadenlaufrichtung vor und/oder hinter dem Strahlenbündel ein Fadenführer angeordnet ist. Hierbei sollte besonders darauf geachtet werden, daß zumindest auf einer der beiden Flanken des Fadens, die auf das ausgesandte oder rückgestrahlte Strahlenbündel hinweisen, der Fadenführer nicht greift, um ein Anschmiegen der abstehenden Filamente an das Garn zu vermeiden.

Die erfindungsgemäße Vorrichtung hat sich besonders bewährt, wenn die Strahlungsquelle ein Lichtleiter ist, an den ein Strahlungssender angeschlossen ist, und /oder wenn der Strahlungsempfänger ein Lichtleiter ist, an den ein Strahlungsmeßkopf angeschlossen ist. Glasfasern als Lichtleiter haben sich hierbei bestens bewährt. Auf diese Weise können Strahlungssender und Strahlungsmeßkopf in sicherer Entfernung von der Fadenlaufstrecke angeordnet werden, sodaß diese abgeschirmt von erhöhten Temperaturen und von Schwingungen, die von den auf den Faden in der Nähe der Meßstelle einwirkenden Fadenbeschleunigungseinheiten ausgehen, angebracht werden können, während die Lichtleiter in der Nähe des Fadens montiert werden können und zwar derart, daß sie den gleichen Schwingungen wie der Faden ausgesetzt sind. Auf diese Weise kann das ausgesandte und das rückgestrahlte Strahlenbündel sehr dicht an dem Faden vorbeigeführt werden. Aufgrund der geringen Ausmaße des Lichtleiters ist auch die Gefahr der Verstaubung deutlich verringert.

Um bei dem Strahlenbündel eine möglichst gebündelte versetzte Umlenkung zu erreichen, ist es vorteilhaft, wenn vor oder an der Eintrittsstelle des ausgesandten Strahlenbündels auf die Mittel zum versetzten Umlenken eine Einrichtung zum Bündeln des Strahlenbündels angeordnet ist. Mittel zum Bündeln eines Strahlenbündels sind hinreichend bekannt. Hierzu eignen sich insbesondere optische Linsen und Blenden.

In einer besonders bevorzugten Ausführungsform der erfindungsgemäßen Vorrichtung ist die Strahlungsquelle und/oder der Strahlungsempfänger zum Faden hin über eine für das Strahlenbündel durchdringbare Platte geschützt. Auf diese Weise können alle Staub- und/oder Temperatur-empfindlichen Vorrichtungsteile in einem Abschirmbehälter untergebracht werden. Der sich auf der von dem Strahlenbündel durchdringbaren Platte sich ablagernde Staub aber auch Ablagerungen wie beispielsweise Präparationsreste können durch geeignete Wahl des Plattenwerkstoffes leicht vermieden zumindest aber leicht wieder entfernt werden. Hierbei muß die Messung, wenn überhaupt, lediglich kurzfristig unterbrochen werden.

Die erfindungsgemäßen Vorrichtung wird anhand der Figuren nachfolgend näher erläutert.

Es zeigen:
- **Figur 1**: eine schematische Darstellung der erfindungsgemäßen Vorrichtung
- **Figur 2**: eine schematische Darstellung des Meßprinzips der erfindungsgemäßen Vorrichtung
- **Figur 3**: eine Ausführungsform der erfindungsgemäßen Vorrichtung im
Querschnitt

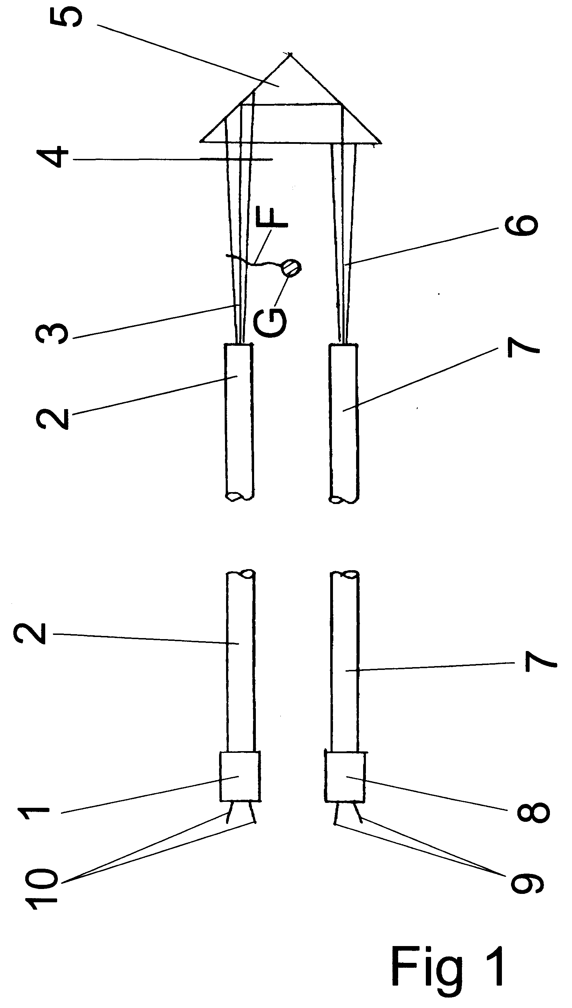

Gemäß Figur 1 ist mit 1 ein Strahlungssender bezeichnet, an den ein Lichtleiter 2 angeschlossen ist. Aus diesem Lichtleiter tritt das vom Strahlungssender kommende Strahlenbündel 3 aus und trifft auf eine Linse 4, wodurch das Strahlenbündel erneut gebündelt wird, wonach das Strahlenbündel in ein Umlenkprisma 5 eintritt. Das Umlenkprisma lenkt das Strahlenbündel zwei mal um sodaß es als versetzt rückgestrahltes Strahlenbündel 6 in Richtung des Lichtleiters 7 in einem Abstand zum ausgesandten Strahlenbündel 3 parallel zu diesem zurückgeführt wird. Der Lichtleiter 7 führt das Strahlenbündel 6 zum Strahlungsmeßkopf 8, dessen Signale über die Leitungen 9 einer Rechnereinheit (nicht dargestellt) zugeführt wird. Der Strahlungssender wird über die Leitungen 10 mit Energie versorgt. Zwischen dem ausgesandten Strahlenbündel 3 und dem versetzt rückgestrahlten Strahlenbündel 6 verläuft das Garn G in einer Richtung, die senkrecht zur Darstellungsebene liegt. Die Elemente der erfindungsgemäßen Vorrichtung sind derart angeordnet, daß der Abstand zwischen Garn G und abgesandtem Strahlenbündel 3 gleich dem Abstand zwischen Garn G und dem versetzt rückgestrahlten Strahlenbündel 6 ist.

Weist das Garn, wie in Figur 1 dargestellt, eine gebrochenes Filament F auf, so steht dieses in der Regel in der in der Figur dargestellten Weise ab und bewirkt beim Durchlaufen durch das Strahlenbündel - in der dargestellten Position das ausgesandte Strahlenbündel 3 - eine Abschattung des Strahlenbündels, welche von dem Strahlungsmeßkopf 8 einer nicht dargestellten Auswerteinheit als Signal über die Leitungen 9 weitergegeben wird. Diese Signale, die durch Abschattungen ausgelöst werden, können von der Auswerteinheit als Zählimpulse zum Zählen der vorhandenen gebrochenen Filamente ausgewertet werden. Je nach Dicke des zu messenden Garns, beträgt der Abstand zwischen Garn G und ausgesandtem Strahlenbündel 3, wie auch der Abstand zwischen Garn G und versetzt rückgestrahltem Strahlenbündel 6 zwischen 3 und 6 mm. Das in Figur 1 dargestellte abstehende Filament F steht nach oben ab, weshalb dieses Filament auch von dem ausgesandten Strahlenbündel erfaßt wird. In gleicher Weise werden auch nach unten abstehende Filamente dann allerdings von dem versetzt rückgestrahlten Strahlenbündel 6 erfaßt, sodaß alle Filamente, die nach oben oder nach unten abstehen, dem Strahlungsmeßkopf als Abschattung gemeldet werden. Es hat sich nun gezeigt, daß diese zweiseitige Messung von abstehenden Filamenten F eine deutlich zuverlässigere Aussage über die Häufigkeit vorhandener Filamentbrüche ergibt als die bisher übliche Messung, die nur abstehende Filamente in einer Richtung registriert.

Sofern eine noch genauere Aussage gewünscht wird, kann die erfindungsgemäße Vorrichtung mehrfach hintereinander angeordnet werden, wobei dann die Ausrichtung der Strahlenbündel 3 und 6 jeweils um einen bestimmten Winkel verdreht angeordnet werden, sodaß dann in mehrere Richtungen abstehende Filamente gemessen werden können.

Da Lichtleiter das Strahlenbündel sicher bis zu einer Länge von 10 m weiterleiten, kann der Strahlungssender und der Strahlungsmeßkopf an einer Stelle untergebracht werden, die von den Bedingungen, die an der Meßstelle herrschen, unabhängig ist. Somit kann die erfindungsgemäße Vorrichtung zum Messen von Ungleichmäßigkeiten am Garn direkt bei der Herstellung oder Weiterverarbeitung des Garnes eingesetzt werden, wo häufig erhöhte Temperaturen herrschen, welche Strahlungssender und insbesondere Strahlungsmeßkopf ungünstig beeinflussen.

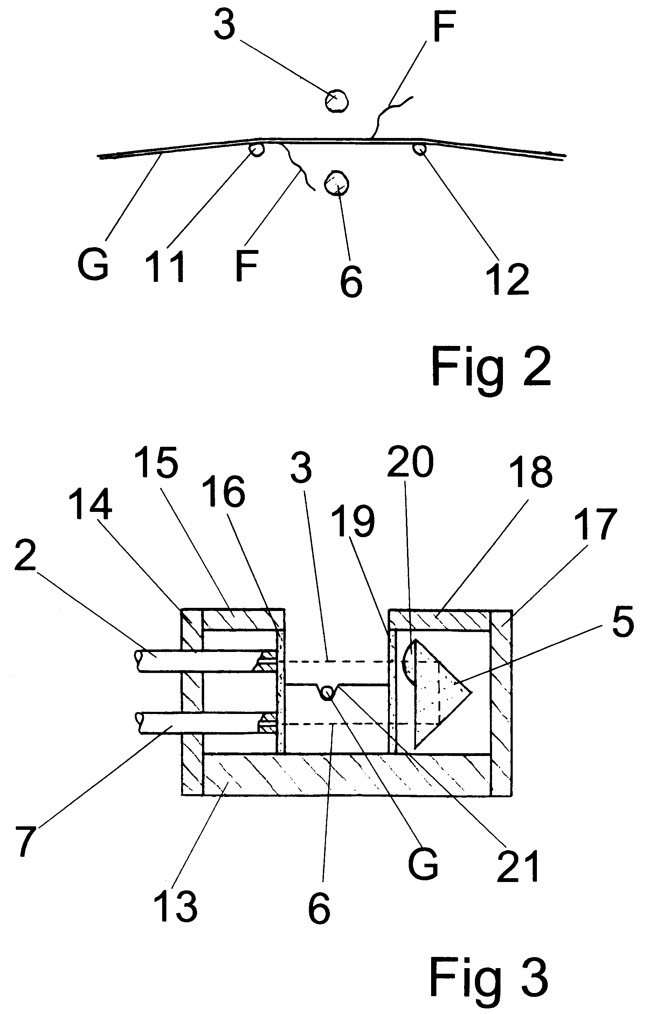

Gemäß Figur 2 wird das von der erfindungsgemäßen Vorrichtung angewandte Meßprinzip dargestellt. In der dargestellten Weise wird das Garn G über einen Garnführer, beispielsweise über einen Stift 11 der Meßstelle zugeführt und nach der Meßstelle über einen weiteren Fadenführer, hier ein Stift 12 von der Meßstelle weggeleitet. Wesentlich hierbei ist, das das Garn G der Meßstelle über die Fadenführer in einer eindeutigen Position gehalten werden. Hierzu wird das Garn G in der dargestellten Ausführungsform von einer tiefer gelegenen Position dem Fadenführer 11 zugeführt und vom Fadenführer 12 einer ebenfalls tiefer gelegenen Position abgeführt. Das ausgesandte und das versetzt rückgestrahlte Strahlenbündel ist hier ebenfalls mit 3 bzw. 6 bezeichnet, wodurch jedoch nicht zum Ausdruck gebracht werden soll, daß diese auch vertauscht angeordnet sein können. In der dargestellten Weise weist das Garn zwei abstehende Filamente F auf, wobei das nach oben stehende Filament F das Strahlenbündel 3 bereits passiert hat, während das nach unten abstehende Filament F noch vor dem Strahlenbündel 6 dargestellt ist und dann eine Abschattung im Strahlenbündel 6 hervorruft, wenn der Faden ein Stück weitergelaufen ist.

In Figur 3 ist eine weitere erfindungsgemäße Vorrichtung dargestellt, bei der Lichtleiter 2 und 7 und Umlenkprisma 5 in einem Gehäuse zu deren Schutz angeordnet sind. Das Gehäuse besteht aus einer Grundplatte 13, an der Seitenwände 14 bzw 17 befestigt sind. An diesen Seitenwänden 14 bzw 17 sind wiederum Abdeckplatten 15 bzw 18 befestigt. Zur Meßstelle hin sind Platten 16 bzw 19 vorgesehen, die die Lichtleiter 2 und 7 bzw. Linse 20 und Umkehrprisma 5 vor äußeren Einflüssen schützen, vom Strahlenbündel jedoch durchdringbar sind. Bei Verwendung von sichtbarem Licht als Strahlenbündel können in einfachsten Falle die Platten 16 und 19 aus Glas oder einem durchsichtigen Kunststoff bestehen.

Somit sind gemäß Figur 3 die Lichtleiter 2 und 7 in einem Gehäuse, welches durch die Grundplatte 13, die Seitenwand 14, die Abdeckplatte 15, die Platte 16 und zwei hinter bzw vor der Zeichenebene angeordnete Vorder- bzw. Rückwände (nicht dargestellt) gebildet ist, geschützt untergebracht. Bei der dargestellten Ausführungsform werden die Lichtleiter 2 und 7 durch die Seitenwand 14 hindurchgeführt. Wie bereits oben ausgeführt, sind diese Lichtleiter in hier nicht dargestellter Weise mit einem Strahlungssender, im einfachsten Falle eine Lampe, bzw. mit einem Strahlungsmeßkopf, im einfachsten Falle ein Photoempfänger, verbunden.

Auch Linse 20 und Umlenkprisma 5 sind in einem Gehäuse, welches durch die Grundplatte 13, die Seitenwand 17, die Abdeckplatte 18, die Platte 19 und zwei hinter bzw. vor der Zeichenebene angeordnete Vorder- bzw. Rückwände (nicht dargestellt) gebildet ist, geschützt untergebracht. Auch hier besteht die Platte 19 aus einem Material, welches von dem Strahlenbündel 3 bzw 6 durchdringbar ist.

Bei dieser Ausführungsform bleibt die Signalübertragung unbeeinflußt von äußeren elektrischen Störungseinflüssen. Selbst elektrische Leitungen, die in der Nähe von Störfelder ausstrahlenden Maschinenteilen vorbeiführen, können selbst dann Signale verfälschen, wenn diese Leitungen abgeschirmt sind. Durch die erfindungsgemäße Anordnung sind solche Verfälschungen wirksam vermieden. Die zur Auswertung der Meßsignale erforderliche störempfindliche Elektronik kann in sicherem Abstand von der Störfeldern der Garnverarbeitungsmaschine untergebracht werden. Der Strahlungsmeßkopf ist nicht den oft an Garnverarbeitungsmaschinen herrschenden erhöhten Temperaturen, die 70 °C und mehr betragen können ausgesetzt. Insofern entfällt auch eine aufwendige Elektronik, über die sonst die Temperatureinflüsse auf den Meßkopf und die Elektronik wieder kompensiert werden müßten. Für die Lichtleiter und die anderen an der Meßstelle befindlichen Teile der erfindungsgemäßen Vorrichtung können Materialien ausgewählt werden, die gegenüber den genannten Temperaturen und gegebenenfalls gegenüber Verschmutzung, die zum Beispiel infolge von Staub und/oder Präparationsnebel oder -spritzern entstehen kann, unempfindlich sind. Hierbei hat sich die Verwendung von Glasfasern beispielsweise mit einem Durchmesser von 0,2 mm, welche mit einem Schutzmantel umgeben sind und dann einen Außendurchmesser von beispielsweise 5 mm aufweisen, als Lichtleiter bestens bewährt.

Zur Messung wird das Garn G über den Fadenführer 21 in Position gehalten. Durch die schlitzförmige Ausbildung des Fadenführers 21 wird das Garn G auch seitlich geführt.

## Patentansprüche

1. Vorrichtung zur optischen Überwachung mindestens eines Fadens auf Unregelmäßigkeiten, insbesondere auf Filamentbrüche von Multifilamentgarnen, mit einer Strahlungsquelle (2), einem Strahlungsempfänger (7) und einem von der Strahlungsquelle (2) ausgesandten und vom Strahlungsempfänger (7) empfangenen quer zum Faden (G) und am Faden in einem definierten Abstand zum Faden vorbeigeführten Strahlenbündel (3), wobei die Strahlungsquelle (2) und der Strahlungsempfänger (7) neben dem Faden angeordnet sind, dadurch gekennzeichnet, daß Strahlungsquelle (2) und Strahlungsempfänger (7) auf derselben Seite des Fadens (G) angeordnet sind, und daß auf der gegenüberliegenden Seite des Fadens (G) Mittel (5) zum versetzten Rückstrahlen des Strahlenbündels derart angeordnet sind, daß das ausgesandte Strahlenbündel (3) auf der einen Seite des Fadens (G) und das versetzt rückgestrahlte Strahlenbündel (6) auf der anderen Seite des Fadens (G) vorbeigeführt wird, wobei das ausgestrahlte Strahlenbündel (3) und das versetzt rückgestrahlte Strahlenbündel (6) möglichst denselben Abstand zum Faden (G) aufweisen und zumindest in etwa parallel zueinander angeordnet sind.

2. Vorrichtung nach Anspruch1, dadurch gekennzeichnet, daß das Mittel zum versetzten Rückstrahlen des Strahlenbündels ein Umlenk-Prisma (5) ist.

3. Vorrichtung nach Anspruch 1 oder 2, dadurch gekennzeichnet, daß das Strahlenbündel ein Laserlichtbündel ist.

4. Vorrichtung nach Anspruch 1 oder 2, dadurch gekennzeichnet, daß das Strahlenbündel ein gebündelter Lichtstrahl ist.

5. Vorrichtung nach Anspruch 4, dadurch gekennzeichnet, daß der Lichtstrahl eine Wellenlänge aufweist, die im nahen infraroten Bereich liegt.

6. Vorrichtung nach einem oder mehreren der Ansprüche 1 bis 5, dadurch gekennzeichnet, daß in Fadenlaufrichtung vor und/oder hinter dem Strahlenbündel ein Fadenführer (21) angeordnet ist.

7. Vorrichtung nach einem oder mehreren der Ansprüche 1 bis 6, dadurch gekennzeichnet, daß die Strahlungsquelle ein Lichtleiter (2) ist, an den ein Strahlungssender (1) angeschlossen ist.

8. Vorrichtung nach einem oder mehreren der Ansprüche 1 bis 7, dadurch gekennzeichnet, daß der Strahlungsempfänger ein Lichtleiter (7) ist, an den ein Strahlungsmeßkopf (8) angeschlossen ist.

9. Vorrichtung nach Anspruch 7 oder 8, dadurch gekennzeichnet, daß der Lichtleiter eine Glasfaser ist.

10. Vorrichtung nach einem oder mehreren der Ansprüche 1 bis 9, dadurch gekennzeichnet, daß vor oder an der Eintrittsstelle des ausgesandten Strahlenbündels (3) auf die Mittel (5) zum versetzten Umlenken eine Einrichtung (4) zum Bündeln des Strahlenbündels (3) angeordnet ist.

11. Vorrichtung nach einem oder mehreren der Ansprüche 1 bis 10, dadurch gekennzeichnet, daß die Strahlungsquelle (2) und/oder der Strahlungsempfänger (7) zum Faden hin über eine für das Strahlenbündel durchdringbare Platte (16) geschützt ist.
